(19) Europäisches Patentamt
European Patent Office
Office européen des brevets

(11) Numéro de publication: **0 297 987**
**A1**

(12) # DEMANDE DE BREVET EUROPEEN

(21) Numéro de dépôt: **88401685.8**

(22) Date de dépôt: **30.06.88**

(51) Int. Cl.[4]: **C 07 D 513/04**
//(C07D513/04,277:00,209:00)

(30) Priorité: **02.07.87 FR 8709376**

(43) Date de publication de la demande:
**04.01.89 Bulletin 89/01**

(84) Etats contractants désignés:
**AT BE CH DE ES FR GB GR IT LI LU NL SE**

(71) Demandeur: **RHONE-POULENC SANTE**
**20, avenue Raymond Aron**
**F-92160 Antony (FR)**

(72) Inventeur: **Rajoharison, Harivelo Gérard**
**18, rue du Tonkin**
**F-69100 Villeurbanne (FR)**

(74) Mandataire: **Le Goff, Yves et al**
**RHONE-POULENC INTERSERVICES Brevets Pharma 25, quai Paul Doumer**
**F-92408 Courbevoie Cédex (FR)**

(54) **Procédé de préparation de l'acide (pyridyl-3)-3 1H,3H-pyrrolo [1,2-c] thiazolecarboxylique-7 dextrogyre.**

(57) Procédé de préparation de l'acide de formule (I) par action d'un ester de l'acide chloro-2 acrylique sur un sel organique de l'acide formyl-3 (pyridyl-3)-2 thiazolidinecarboxylique-4 cis ou en majorité cis en présence d'un chlorure d'acide et de triéthylamine puis saponifie l'ester de formule (VII) obtenu.

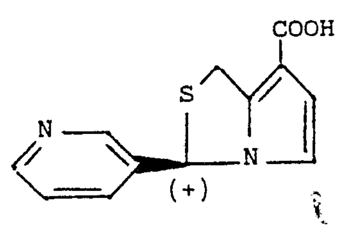

(I)

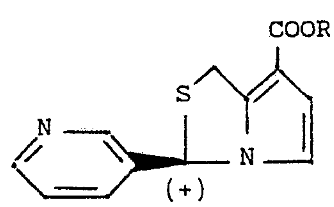

(VII)



Bundesdruckerei Berlin

## Description

### PROCEDE DE PREPARATION DE L'ACIDE (PYRIDYL-3)-3 1H,3H-PYRROLO [1,2-c] THIAZOLECARBOXYLIQUE-7 DEXTROGYRE

La présente invention concerne un nouveau procédé de préparation de l'acide (pyridyl-3)-3 1H,3H-pyrrolo [1,2-c] thiazolecarboxylique-7 dextrogyre de formule :

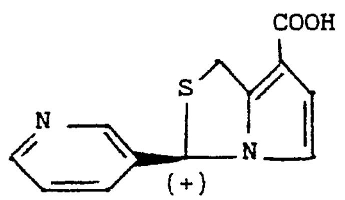

(I)

utile comme intermédiaire dans la préparation de médicaments destinés à traiter les affections dans lesquelles est impliqué le rôle physiologique du PAF-acéther, notamment pour soigner les allergies, l'inflammation et empêcher l'agrégation des plaquettes de sang. De tels produits sont connus par le brevet européen n° 0 115 979.

Jusqu'à ce jour, l'acide de formule (I) était préparé à partir de l'acide racémique correspondant par dédoublement au moyen d'une base optiquement active telle que la (+)α-méthylbenzylamine [(+)α-MBA], c'est-à-dire selon les méthodes classiques. L'acide (pyridyl-3)-3 1H,3H-pyrrolo [1,2-c] thiazolecarboxylique-7 racémique était préparé selon le procédé décrit dans le brevet européen n° 0 115 979 à partir de l'acide formyl-3 (pyridyl-3)-2 thiazolidinecarboxylique-4 selon le schéma suivant :

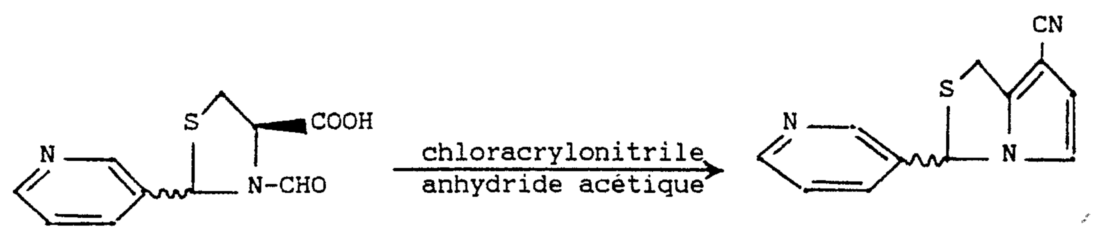


mélange de produits cis et trans (II)

mélange de produits dextrogyre et lévogyre (III)

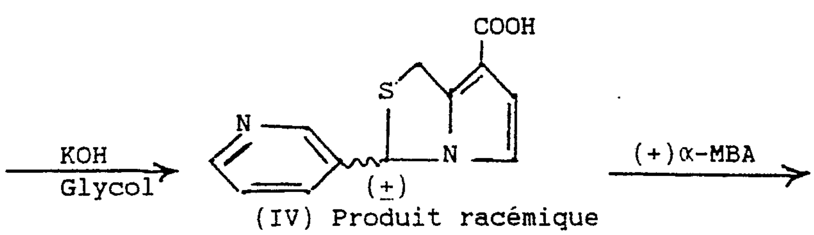


(IV) Produit racémique

(I)

Cette synthèse présente trois inconvénients principaux :

- Quel que soit le rapport des formes cis et trans du produit formylé de formule (II), on n'obtient jamais de rendements supérieurs à 36% en nitrile de formule (III),
- La condensation de chloracrylonitrile sur l'acide de formule (II) nécessite 5 équivalents de chloracrylonitrile par mole d'acide, ce qui entraîne l'obligation de récupérer et de recycler le réactif lorsqu'on envisage un procédé à l'échelle industrielle,
- Quel que soit le rapport des formes dextrogyre et lévogyre du nitrile du formule (III), les conditions sévères de saponification conduisent à l'obtention d'un acide (IV) racémique, ce qui implique nécessairement une séparation ultérieure par dédoublement au moyen d'une base optiquement active et entraîne de nombreuses recristallisations et une baisse des rendements; De ce fait, l'ensemble de la synthèse ne permet pas de

préparer l'acide de formule (I) avec un rendement supérieur à 10% environ à partir de l'acide formylé de formule (II).

Ces inconvénients ont été supprimés ou atténués en mettant en oeuvre un nouveau procédé qui apporte à la synthèse précédente les modifications fondamentales suivantes :

- Utilisation d'un acide formyl-3 (pyridyl-3)-2 thiazolidinecarboxylique-4 de forme cis ou en majorité cis,
- Remplacement du chloracrylonitrile par un ester de l'acide chloro-2 acrylique conduisant ainsi à l'obtention d'un ester facilement saponifiable dans des conditions non racémisantes,
- Remplacement de l'anhydride acétique par un chlorure d'acide dans la réaction de condensation avec l'acide formylé et emploi un solvant à bas point d'ébullition permettant d'effectuer la réaction dans des conditions douces, avec des rendements améliorés, en évitant la formation de goudrons.

Ainsi, le procédé selon la présente invention consiste à faire réagir un ester de l'acide chloro-2 acrylique de formule générale :

$$CH_2 = \underset{Cl}{C} - COOR \qquad (V)$$

dans laquelle R représente un radical alcoyle contenant 1 à 4 atomes de carbone en chaîne droite ou ramifiée ou radical benzyle ou aryle sur un sel de base organique de l'acide formyl-3 (pyridyl-3)-2 thiazolidinecarboxylique-4 (2R, 4R) de formule :

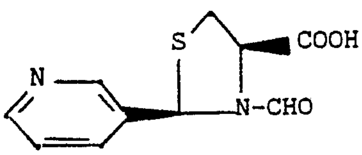

(VI)

en présence d'un chlorure d'acide.

Sans que cela soit limitatif, il est particulièrement avantageux d'utiliser comme chlorure d'acide le chlorure de paratoluène sulfonyle, le chlorure de méthanesulfonyle ou le chlorure d'oxalyle, pour obtenir un ester dextrogyre de formule générale :

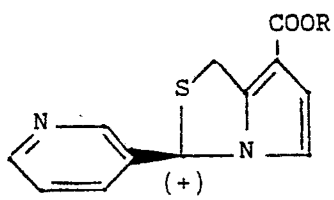

(VII)

dans laquelle R est défini comme précédemment, puis à saponifier l'ester (VII) dans des conditions douces non racémisantes.

Dans la pratique, on opère généralement dans un solvant organique chloré capable de dissoudre le sel de l'acide (VI), tel que le dichlorométhane ou le dichloroéthane à une température comprise entre 20°C et la température de reflux du mélange réactionnel.

Comme base organique pour la formation du sel de l'acide de formule (VI), on peut employer une trialcoylamine telle que la triéthylamine, la tributylamine ou la pyridine.

Selon une variante de l'invention, on prépare, sans l'isoler, l'ester de l'acide chloro-2 acrylique de formule générale (V) par action d'une base organique sur l'ester de l'acide dichloro-1,2 propionique correspondant de formule générale :

$$ClCH_2 - \underset{Cl}{CH} - COOR \qquad (VIII)$$

dans laquelle R a la définition donnée précédemment pour l'ester de formule (V).

Comme base organique, on emploie généralement la base qui sert à former le sel de l'acide de formule (VI) comme il a été dit précédemment, de préférence la triéthylamine.

On opère généralement dans un solvant organique tel que celui qui sert à la condensation de l'ester de formule (V) sur l'acide de formule (VI), de préférence le dichlorométhane ou le dichloro-1,2 éthane.

La saponification de l'ester de formule générale (VII) peut être effectuée par toute méthode connue de l'homme du métier, susceptible de transformer un ester en acide dans des conditions douces non racémisantes. Il est particulièrement avantageux d'effectuer la saponification par action d'un hydroxyde de métal alcalin tel que la soude ou la potasse dans un mélange hydroalcoolique à une température comprise entre 25 et 45°C.

L'acide de formule (VI) peut être préparé selon la méthode décrite dans le brevet européen n° 0 115 979 suivie d'une recristallisation pour obtenir l'acide de forme cis.

L'acide de formule (I) ainsi que les intermédiaires de synthèse peuvent être purifiés par les moyens habituels connus de l'homme du métier, par exemple par chromatographie, recristallisation ou distillation.

On connait déjà des réactions de cycloadditions conduisant à des pyrrolothiazoles optiquemnt actifs [H.T. NAGAJAWA, D.J.W. GOON et F.N. SHIRDTA, J. Heterocyclic Chem., 18, 1047 (1981)]. Les réactions décrites dans cette publication ont été effectuées sur des substrats différents et selon des conditions différentes (emploi d'anhydride acétique et chauffage à 100-105° C). Les esters de l'acide chloro-2 acrylique (notamment le chloro-2 acrylate d'éthyle), n'avaient jamais été employés pour la préparation de pyrrolothiazoles. Il n'était donc pas évident que ceux-ci conduiraient à l'acide de formule (I) de façon stéréosélective. De plus, la transposition des conditions de NAGASAWA et col. mentionné ci-dessus au cas du chloro-2 acrylate d'éthyle et de l'acide formyl-3 (pyridyl-3)-2 thiazolidinecarboxylique-4 conduit à des rendements faibles [de l'ordre de 20% à partir de l'acide de formule (VI)]. La faiblesse des rendements est due principalement à la formation importante de goudrons.

Ainsi, la présente invention apporte de façon inattendue une solution au problème cherché, à savoir la préparation de l'acide de formule (I) avec des rendements améliorés, sans avoir de séparation de diastéréoisomères.

Les exemples suivants montrent de façon plus détaillée, comment dans la pratique l'invention peut être réalisée.

## EXEMPLE 1

### a) Préparation de l'ester (formule (VII) avec R = $C_2H_5$) en présence de chlorure de tosyle

Dans un réacteur de 100 litres, on charge 8169 g de chlorure de tosyle, 16,5 litres de dichlorométhane et 7781 g de dichloro-2,3 propionate d'éthyle. On porte ce mélange à reflux (40-42° C) puis on ajoute en 1 heure 45 minutes une solution prélablement préparée de 9350 g du diastéréoisomère cis de l'acide formyl-3 (pyridyl-3)-2 thiazolidinecarboxylique-4 dans 13,8 litres de dichlorométhane et 4375 g de triéthylamine. Après 20 minutes d'agitation supplémentaire, on ajoute en 1 heure et 15 minutes 13126 g de triéthylamine. On laisse agiter à reflux (40-42° C) pendant une heure et demie. On refroidit le mélange réactionnel à 20° C puis on reprend le mélange réactionnel avec 12 litres d'eau distillée. la phase organique est décantée, lavée 3 fois avec de l'eau distillée (20 litres au total) puis séchée sur 6 kg de sulfate de sodium. Après filtration et évaporation du solvant, on obtient 14030 g de mélange brut contenant 8135 g (dosage CLHP, titre 58%) de (pyridyl-3)-3 1H,3H-pyrrolo [1,2-c] thiazolecarboxylate-7 d'éthyle (rendement = 75,6% par rapport à la thiazolidine engagée).

La pureté énantiomérique du produit obtenu est supérieure à 99,4% en énantiomère d par analyse CLHP sur colonne chirale Pirkle type 1-A (éluant hexane/isopropanol : 90/10, débit 0,3 ml/mn, P = 4 bars).

### b) Préparation de l'acide de formule (I)

On dissout 84,35 g de l'ester brut obtenu précédemment dans 134 cm3 d'éthanol et on ajoute à 25° C en 20 minutes une solution contenant 35,3 g de potasse en pastilles et 214 cm3 d'eau. On porte le mélange à 40° C et maintient cette température pendant 18 heures. On ajoute ensuite 8,34 g de noir décolorant puis on continue le chauffage à 40° C pendant 2 heures supplémentaires. On filtre à chaud en présence de terre de diatomée. Après refroidissement à 20° C, le filtrat est amené à pH 4 par addition de 40 cm3 d'acide chlorhydrique concentré. Les cristaux apparus sont séparés par filtration puis lavés trois fois à l'eau puis séchés sous pression réduite (20 mm de mercure; 2,7 kPa) à 20° C. On obtient ainsi 41,9 g (rendement 95,3%) d'acide (pyridyl-3)-3 1H,3H-pyrrolo [1,2-c] thiazolecarboxylique-7 (titre 93%) sous forme de cristaux de couleur ocre fondant à 214° C et présentant un pouvoir rotatoire = +151° (c = 2, NaOH N). Le rendement global est alors de 72% par rapport à l'acide formyl-3 (pyridyl-3)-2 thiazolidinecarboxylique-4 mis en oeuvre.

L'analyse RMN 1H en présence de 1(-)a-méthylbenzylamine ne permet d'observer que le sel de l'énantiomère d de l'acide décrit (pureté énantiomérique >95%).

## EXEMPLE 2

### a) Préparation de l'ester (formule (VII) avec R = $C_2H_5$) en présence de chlorure de tosyle

Dans un réacteur de 100 litres, on charge 9562 g de chlorure de paratoluènesulfonyle, 20 litres de dichlorométhane et 9108 g de dichloro-2,3 propionate d'éthyle à 95% de pureté. On chauffe le mélange au reflux du dichlorométhane (40° C) puis on ajoute en 1 heure à cette température le mélange préformé de 10990 g d'acide formyl-3 (pyridyl-3)-2 thiazolidinecarboxylique-4 cis, de 5120 g de triéthylamine et de 16,2 litres de dichlorométhane. On laisse encore agiter pendant 15 minutes puis on ajoute en une heure et demie 21,336 litres de triéthylamine à la température de reflux du dichlorométhane. La réaction est prolongée pendant encore une heure à cette température. Après refroidissement à température ambiante, on reprend le mélange réactionnel par 15 litres d'eau, décante la phase organique et extrait la phase aqueuse par 2 fois 6 litres de

dichlorométhane. Les extraits organiques sont rassemblés puis lavés 3 fois par 24 litres d'eau au total. Après évaporation du solvant, on obtient 15028 g d'une huile brune qui cristallise au repos. l'analyse CLHP de ce produit permet de doser 10670 g de (pyridyl-3)-3 1H, 3H-pyrrolo [1,2-c] thiazole carboxylate d'éthyle, soit un rendement de 84,6% par rapport à l'acide engagé.

b) Purification de l'ester obtenu

On dissout 114 g de l'ester brut obtenu précédemment dans 470 cm3 d'éthanol. On porte le mélange à reflux puis on ajoute 10 g de noir décolorant. On laisse agiter à reflux pendant une heure et demie. Après refroidissement à température ambiante, on filtre sur terre de diatomée. Le solvant est évaporé et le résidu est traité par 275 g de silice (0,063-0,2 mm) en présence de 600 cm3 de dichlorométhane. Après évaporation du solvant, on agite le résidu en présence de 100 cm3 de pentane. Le produit précipite. On obtient ainsi 69,3 g de (pyridyl-3)-3 1H,3H-pyrrolo [1,2-c] thiazolecarboxylate d'éthyle sous forme d'une poudre de couleur jaune clair et fondant à 64°C (titre CLHP - 95,5%).

c) Préparation de l'acide de formule (I)

A une solution de 40 g de l'ester purifié précédemment dans 110 cm3 d'éthanol, on ajoute en 10 minutes une solution de 29 g de potasse dans 175 cm3 d'eau distillée. On chauffe le mélange à 40°C pendant 17 heures, puis on ajoute 4 g de noir décolorant. Après 2 heures de chauffage supplémentaires à 40°C, on refroidit à 20°C puis on filtre sur terre de diatomée. On amène le mélange à pH 4 par addition de 30 cm3 d'acide chlorhydrique concentré (37%). Les cristaux apparus sont séparés par filtration, lavés trois fois à l'eau puis séchés sous pression réduite (20 mm de mercure; 2,7 kPa) à 20°C. On obtient ainsi 31,4 g d'acide (pyridyl-3)-3 1H,3H-pyrrolo [1,2-c] thiazolecarboxylique-7 sous forme de cristaux de couleur crème fondant à 215°C. L'analyse RMN 1H du produit obtenu en présence de 1(-)α-méthylbenzylamine ne permet d'observer que le sel de l'énantiomère d de l'acide décrit (pureté énantiomère >95%). Le rendement de la réaction est de 88% par rapport à l'ester engagé soit un rendement global de 63,6% par rapport à l 'acide formyl-3 thiazolidinecarboxylique-4.

EXEMPLE 3

a) Préparation de l'ester (formule (VII) avec R = $C_2H_5$) en présence de chlorure de méthanesulfonyle

A une solution agitée de 3,54 cm3 de chlorure de méthanesulfonyle dans 47 cm3 de dichlorométhane, on ajoute en une heure à une température voisine de 20°C une solution contenant 10 g du diastéréoisomère cis de l'acide formyl-3 (pyridyl-3)-2 thiazolidinecarboxylique-4, de 6,45 cm3 de triéthylamine et 38,5 cm3 de dichlorométhane. On laisse agiter pendant 15 minutes puis on ajoute en 35 minutes à 20°C une solution contenant 6,21 g de chloro-2 acrylate d'éthyle, 13 cm3 de triéthylamine et 21 cm3 de dichlorométhane. On laisse agiter pendant 30 minutes puis on porte à reflux (40°C) pendant 30 minutes. On refroidit ensuite le mélange à 20°C puis on reprend le mélange réactionnel avec 100 cm3 d'eau distillée. La phase aqueuse est extriate avec 25 cm3 de dichlorométhane et la couche organique lavée avec une solution aqueuse demi-saturée de chlorure de sodium. Après séchage avec du sulfate de sodium, filtration et évaporation du solvant, on obtient 10,75 g de produit brut. Ce dernier est purifié par chromatographie liquide sur une colonne de 50 mm de diamètre contenant 100 g de silice (0,063-0,2 mm) en éluant avec de l'acétate d'éthyle. On obtient ainsi 7 g de (pyridyl-3)-3 1H,3H-pyrrolo [1,2,-c] thiazolecarboxylate-7 d'éthyle pur sous forme d'une huile jaune qui cristallise au repos (rendement 60,8% par rapport à l'acide engagé).

b) Préparation de l'acide de formule (I)

A une solution de 6,13 g de l'ester obtenu précédemment dans 17 cm3 d'éthanol, on ajoute en 10 minutes une solution contenant 4,43 g de potasse et 27 cm3 d'eau distillée. On chauffe le mélange à 40°C pendant 18 heures puis on ajoute 0,617 g de noir décolorant. Après 1 heure supplémentaire à 40°C, on refroidit à 20°C puis on filtre. On amène le mélange à pH 4 par addition d'acide chlorhydrique concentré. Les cristaux apparus sont séparés par filtration puis lavés trois fois à l'eau et séchés sous pression réduite (20 mm de mercure; 2,7 kPa) à 20°C. On obtient ainsi 4,55 g d'acide (pyridyl-3)-3 1H,3H-pyrrolo [1,2-c] thiazolecarboxylique-7 sous forme de cristaux de couleur crème fondant à 214°C et présentant un pouvoir rotatoire = +158° (c = 2; NaOH N) soit un rendement global de 50,3% par rapport à l'acide formyl-3 (pyridyl)-2 thiazolidinecarboxylique-4 mis en oeuvre.

EXEMPLE 4

a) Préparation de l'ester (formule (VII) avec R = $C_2H_5$) en présence de chlorure d'oxalyle

Dans un tétracol de 250 cm3 muni d'une agitation mécanique, d'une ampoule de coulée, d'un thermomètre et d'un réfrigérant relié à un gazomètre, on charge 10 g du diastéréoisomère cis de l'acide formyl-3 (pyridyl-3)-2 thiazolidinecarboxylique-4 et 63,5 cm3 de dichlorométhane. On ajoute ensuite goutte à goutte en 14 minutes à température ambiante 6,45 cm3 de triéthylamine. Le milieu devient homogène après 10 minutes d'agitation. On ajoute alors en 48 minutes à 24° C une solution de 5,90 g de chlorure d'oxalyle dans 22 cm3 de dichlorométhane. Au cours de l'addition, le milieu devient progressivement hétérogène de couleur jaune orangé avec dégagement gazeux de monoxyde de carbone et de dioxyde de carbone. On laisse agiter pendant encore 15 minutes à température ambiante puis on ajoute en 34 minutes une solution contenant 6,20 g de chloro-2 acrylate d'éthyle, 9,44 g de triéthylamine et 21 cm3 de dichlorométhane. On observe à nouveau un dégagement de dioxyde de carbone; le milieu devient progressivement homogène de couleur marron. On chauffe ensuite le mélange réactionnel à 40° C pendant 30 minutes. On refroidit à 20° C et reprend le mélange par 100 cm3 d'eau. On décante la phase organique puis on extrait la phase aqueuse par 100 cm3 de dichlorométhane et sèche les extraits organiques sur du sulfate de sodium. Après évaporation du solvant sous pression réduite (20 mm de mercure; 2,7 kPa) à 20° C, on obtient 11 g de produit brut. Ce dernier est purifié par chromatographie liquide sur une colone de 50 mm de diamètre contenant 100 g de silice (0,063-0,2 mm) en éluant avec de l'acétate d'éthyle. On obtient ainsi 5,78 g de (pyridyl-3)-3 1H,3H-pyrrolo [1,2-c] thiazolecarboxylate-7 d'éthyle sur sous forme d'une huile jaune qui cristallise au repos (rendement 50,2% par rapport à l'acide engagé).

b) Préparation de l'acide de formule (I)

A une solution de 5,26 g de l'ester obtenu précédemment, dans 14,6 cm3 d'éthanol, on ajoute en 10 minutes une solution contenant 3,8 g de potasse et 25 cm3 d'eau distillée. On chauffe le mélange à 40°C pendant 18 heures. On ajoute alors 0,526 g de noir décolorant. Après une heure de chauffage supplémentaire à 40°C, on refroidit à 20°C puis on filtre. On amène ensuite le mélange à pH 4 par addition d'acide chlorhydrique concentré. Les cristaux apparus sont séparés par filtration, puis lavés trois fois à l'eau et séchés sous pression réduite (20 mm de mercure, 2,7 kPa) à 20° C. On obtient ainsi 4,32 g d'acide (pyridyl-3)-3 1H,3H-pyrrolo [1,2-c] thiazolecarboxylique-7 sous forme de cristaux de couleur crème fondant à 215° C et présentant un pouvoir rotatoire = +159,7° (c = 2, NaOH N) soit un rendement global de 45,9% par rapport à l'acide formyl-3 (pyridyl-3)-2 thiazolidinecarboxylique-4 mis en oeuvre.

**Revendications**

1 - Procédé de préparation de l'acide (pyridyl-3)-3 1H,3H-pyrrolo [1,2-c] thiazolecarboxylique-7 dextrogyre de formule :

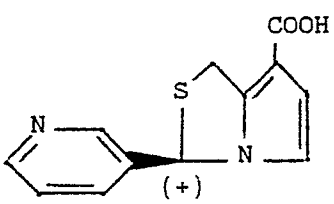

(I)

caractérisé en ce que l'on fait réagir un ester de l'acide chloro-2 acrylique de formule générale :

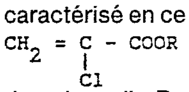

(V)

dans laquelle R représente un radical alcoyle contenant 1 à 4 atomes de carbone en chaîne droite ou ramifiée ou un radical benzyle ou aryle sur un sel organique de l'acide formyl-3 (pyridyl-3)-2 thiazolidinecarboxylique-4 cis ou en majorité cis de formule :

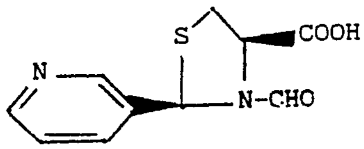

(VI)

en présence d'un chlorure d'acide et de triéthylamine pour obtenir un ester de formule générale :

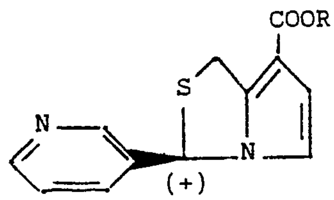

(VII)

dans laquelle R est défini comme précédemment dans l'ester de formule (V), puis on saponifie ledit ester de formule générale (VII) dans des conditions douces non racémisantes, et isole le produit obtenu.

2 - Procédé selon la revendication 1, caractérisé en ce que le chlorure d'acide employé par condenser l'ester de formule (V) sur le sel organique de l'acide de formule (VI) est le chlorure de paratoluènesulfonyle.

3 - Procédé selon la revendication 1, caractérisé en ce que le chlorure d'acide employé pour condenser l'ester de formule (V) sur le sel organique de l'acide de formule (VI) est le chlorure de méthanesulfonyle.

4 - Procédé selon la revendication 1, caractérisé en ce que le chlorure d'acide employé pour condenser l'ester de formule (V) sur le sel organique de l'acide de formule (VI) est le chlorure d'oxalyle.

5 - Procédé selon la revendication 1, caractérisé en ce que la base employée pour préparer le sel organique de l'acide de formule (VI) est la triéthylamine.

6 - Procédé selon la revendication 1, caractérisé en ce que l'ester de l'acide chloro-2 acrylique est le chloro-2 acrylate d'éthyle.

7 - Procédé selon la revendication 1, caractérisé en ce que la saponification de l'ester de formule générale (VII) s'effectue au moyen d'une solution hydroalcoolique de potasse entre 25 et 45° C.

8 - Procédé selon la revendication 1, caractérisé en ce que l'ester de l'acide chloro-2 acrylique de formule générale (V) est préparé in situ par action d'une base sur un ester de l'acide dichloro-2,3 propionique de formule :

$$ClCH_2 - \underset{\substack{|\\ Cl}}{CH} - COOR \qquad (VIII)$$

dans laquelle R représente un radical alcoyle contenant 1 à 4 atomes de carbone en chaîne droite ou ramifiée ou un radical benzyle ou aryle.

9 - Procédé selon la revendication 8, caractérisé en ce que la base utilisée pour préparer l'ester de l'acide chloro-2 acrylique est la triéthylamine.

Office européen des brevets

# RAPPORT DE RECHERCHE EUROPEENNE

Numero de la demande

EP 88 40 1685

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | EP-A-0 115 979 (RHONE-POULENC)<br>* Revendication 2 * | 1 | C 07 D 513/04 //<br>(C 07 D 513/04<br>C 07 D 277:00<br>C 07 D 209:00 ) |
| P,X | EP-A-0 252 823 (RHONE-POULENC)<br>* Revendication 3 * | 1 | |
| | | | **DOMAINES TECHNIQUES RECHERCHES (Int. Cl.4)** |
| | | | C 07 D 513/00 |

Le présent rapport a été établi pour toutes les revendications

| Lieu de la recherche | Date d'achèvement de la recherche | Examinateur |
|---|---|---|
| LA HAYE | 29-09-1988 | ALFARO I. |

### CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)